Europäisches Patentamt

(19) European Patent Office

Office européen des brevets

(11) **EP 0 601 886 B1**

(12) **EUROPEAN PATENT SPECIFICATION**

(45) Date of publication and mention
of the grant of the patent:
**26.02.1997 Bulletin 1997/09**

(51) Int. Cl.$^6$: **C07C 1/04**

(21) Application number: **93310000.0**

(22) Date of filing: **10.12.1993**

(54) **Manufacture of organic liquids**

Verfahren zur Herstellung von organischen Flüssigkeiten

Procédé pour la préparation de liquides organiques

(84) Designated Contracting States:
**DE ES FR GB IT NL**

(30) Priority: **10.12.1992 US 988882**

(43) Date of publication of application:
**15.06.1994 Bulletin 1994/24**

(73) Proprietor: **FOSTER WHEELER ENERGY LIMITED**
**Reading Berkshire, RG1 1LX (GB)**

(72) Inventor: **Skinner, Geoff**
**Berkshire, RG1 1LX (GB)**

(74) Representative: **Rackham, Anthony Charles et al**
**Lloyd Wise, Tregear & Co.,**
**Commonwealth House,**
**1-19 New Oxford Street**
**London WC1A 1LW (GB)**

(56) References cited:
**US-A- 4 383 837**          **US-A- 4 833 170**

Printed by Rank Xerox (UK) Business Services
2.13.14/3.4

# Description

This invention relates to the manufacture of organic liquids, by which it is meant compounds which (1) are liquids at atmospheric pressure and temperature and (2) are composed of carbon, hydrogen and optionally oxygen. In particular this invention relates to the manufacture of organic liquids from gases containing hydrogen, nitrogen, and carbon monoxide. More particularly, the invention relates to processes in which the synthesis gas is manufactured by reaction of a hydrocarbon or other carbonaceous feed material with air or oxygen-enriched air, such that the synthesis gas contains a significant proportion of nitrogen.

Methods of producing organic liquids from mixtures of hydrogen and carbon monoxide (commonly called synthesis gas) are well known in the art. Methanol, for instance, is normally manufactured by reacting carbon oxides (mainly carbon monoxide) with hydrogen over a copper containing catalyst, whereby the carbon oxides and hydrogen are converted to methanol.

Another well-known process that uses synthesis gas is the Fischer Tropsch system, which is used to manufacture synthetic transportation fuels (gasoline and diesel).

Higher alcohols and olefins are among other products that may be manufactured from synthesis gas.

To achieve a more complete conversion of the synthesis gas into products, the effluent gas from the organic synthesis reactor is usually recycled to the reactor inlet, thus forming the well-known 'synthesis loop'. If the synthesis gas used in such a loop process is made by the partial combustion of a hydrocarbon or other carbonaceous material with air, the nitrogen present in the air will build-up to undesirable levels in the synthesis loop.

In order to synthesize organic liquids without an undesirable build-up of nitrogen in the synthesis loop, the nitrogen has either to be removed from the synthesis gas or the partial combustion process has to be accomplished in the absence of air, i.e., with oxygen.

The concept and practice of synthesizing hydrocarbon materials, particularly gasoline and diesel fuel, from hydrogen and carbon monoxide is well known. Typically, but not exclusively, the reaction proceeds according to the expression:

$$(2n + 1) \, H_2 + nCO \rightarrow C_nH_{(2n + 2)} + nH_2O$$

this requiring a synthesis gas having an $H_2/CO$ molar ratio of approximately 2.

Products containing carbon, hydrogen and oxygen, for example methanol or higher alcohols, may also be synthesized from hydrogen and carbon monoxide, typically according to the expression

$$2n \, H_2 + n \, CO \rightarrow c_nH_{(2n + 1)} \, OH + (n - 1) \, H_2O$$

Synthesis gases for the above-mentioned processes may be produced by reaction of a feedstock such as natural gas, heavy oil or coal with an oxygen-containing gas.

The use of oxygen-enriched air or air alone as the oxidant gas reduces or eliminates the cost of an air separation (oxygen) plant.

With such a once-through arrangement, it is typically difficult to achieve an overall conversion of $H_2$ and CO into the desired products in excess of 90% or so. Consequently the tail-gas from the once-through process has a significant content of combustible gases. These gases consist of unconverted $H_2$ and CO plus light hydrocarbon gases, both originating in the synthesis gas production system and formed as by-products of the synthesis itself.

In order to dispose of the combustible content of the nitrogen-rich tail gas in an economical and environmentally acceptable way, it has been proposed to burn the gas in a boiler or a gas turbine by Garwood et al (U.S. Patent No. 4,549,396), or in a gas turbine integrated with the compression plant supplying the oxidizing gas to the process.

The combustion of the tail gas in a boiler or turbine is within the scope of the invention but it depends on there existing in the locality a demand for steam or electric power, since generally the total combustible heat content of the tail gas substantially exceeds the heat and power requirements of the synthetic fuels plant itself.

One objective of the present invention therefore is to provide a means of recycling most of the combustible content of the nitrogen-rich tail gas within the synthesis plant itself, thereby avoiding dependence on an external demand for heat or power.

As described in European Patent No. 261 771 organic liquids, such as methanol, can be synthesised from gases derived from the partial combustion of hydrocarbons or other carbonaceous materials with air, without the undesirable build-up of nitrogen occurring within the system by passing the gases through one or more organic liquid synthesis reactors and condensing and removing organic liquid, e.g. methanol, from the effluent stream from each of the reactors.

According to the present invention there is provided a process for manufacturing an organic liquid which is methanol, ethanol or a higher alcohol, a Fischer Tropsch product or an olefin, comprising:

(a) forming a gas comprising hydrogen, nitrogen and carbon monoxide by the partial oxidation of a hydrocarbon or other carbonaceous material with air,
(b) passing the said gas without the removal of the nitrogen through at least one organic liquid synthesis reactor,
(c) condensing and removing the organic liquid to leave a first product gas,
(d) separating carbon dioxide from the first product gas to obtain a second product gas,

(e) methanating the second product gas to obtain a third product gas which is substantially a gas free of carbon oxides, and

(f) separating the hydrocarbons from the third product gas to obtain a nitrogen-rich waste gas and recycling the hydrocarbon rich gas to step a for forming a gas comprising hydrogen, nitrogen and carbon monoxide.

Thus the carbon monoxide and some of the carbon dioxide in the tail gas is converted into methane by reaction with the hydrogen present ("methanation"), the methanated gas is separated into a methane-rich stream, which is recycled to the synthesis gas production section, and an almost combustible-free nitrogen waste stream. The waste nitrogen may be heated prior to expansion to atmospheric pressure through a turbine.

By this arrangement, nearly all the combustible material in the tail gas from the synthesis unit, comprising not only the unconverted hydrogen and carbon monoxide but also residual methane from the synthesis gas production and hydrocarbon gases formed in the synthesis section itself, can be recovered with only a small loss of energy.

The physical separator, e.g., for separating methane, can be a cryogenic separator, in which case it will be necessary to remove sufficient carbon dioxide upstream of the methanator to ensure that the methanated gas is essentially free of carbon dioxide. If a carbon dioxide removal plant is needed in any event in order to recycle some carbon dioxide to the synthesis gas production section, the incremental cost of providing extra carbon dioxide removal capacity necessary for practice of the invention would be small.

The synthesis gases can be produced by a partial combustion of hydrocarbons, such as natural gas or petroleum, or other carbonaceous material, such as coal or coke, with air and oxygen-enriched air. Steam can also be present during the combustion process.

Any appropriate process can be used for partial combustion. An example of a process for the partial combustion of hydrocarbons is the Texaco partial oxidation process which operates at around 1400°C and between $9.8 \times 10^5$ and $9.8 \times 10^6 N/m^2$ (10 and 100 atmospheres). An example of a process for the partial combustion (i.e. gasification) of coal is the U-gas fluid bed process, which operates at around 1000°C and at $4.9 \times 10^5$ to $4.9 \times 10^6 N/m^2$ (5 to 50 atmospheres).

Whichever process is used for the partial combustion operation, the synthesis gas produced will have as its main constituents hydrogen, carbon monoxide and nitrogen.

For the efficient operation of the organic synthesis, it may be necessary for the synthesis gas to be essentially free of sulphur compounds, carbon dioxide and/or water vapour.

Sulphur compounds, if present in the feedstock, can be removed either from the feedstock before partial combustion or afterwards. In the case of a hydrocarbon gas feedstock containing a few parts per million of sulphur compounds, this would be best accomplished upstream of the partial combustion using an absorbent such as zinc oxide operating at around 400°C. In the case of a hydrocarbon gas with a large sulphur content (over 500 ppm), or a coal, coke, or petroleum oil, the removal of sulphur is best accomplished by washing the partial combustion product gas with a solvent, such as monoethanolamine solution.

Carbon dioxide can be removed from the partial combustion product gas by a similar monoethanolamine process or other established washing process. Water vapour can be removed from the partial combustion product gas by cooling and condensation.

The resulting purified gas stream comprising mainly hydrogen, carbon monoxide and nitrogen is passed through a series of one or more reactors wherein the organic synthesis desired is performed.

Any number of synthesis reactors can be used with the scope of this invention. The number of reactors used in the organic liquid synthesis generally ranges from one to about four, depending on the liquid produced.

Any catalyst for synthesizing organic liquids can be used in this invention. Typical catalysts include copper and zinc-containing catalysts for methanol manufacture and iron oxide-based catalysts for Fischer Tropsch processing.

The organic liquid synthesis reactors generally operate at temperatures and pressures suitable to the catalyst being used. Typically, the synthesis reactor will operate at temperatures of from 100 to 400°C and a pressure of from atmospheric to $3.9 \times 10^6 N/m^2$ (40 atmospheres), or at temperatures of from 200 to 500°C and a pressure of from atmospheric to $9.8 \times 10^6 N/m^2$ (100 atmospheres) in the presence of a catalyst comprising copper, iron and zinc compounds as the principal active constituents.

Preferably one or more (e.g. two to four) organic liquid synthesis reactors arranged in series are used as described in the above noted European Patent No. 261 771 the organic product is removed from the effluent stream from each reactor. This separation is generally carried out by cooling and condensing the organic product from the effluent. The condensation process may take place at a temperature of from -100°C to 150°C and a pressure of from atmospheric pressure to $9.8 \times 10^6 N/m^2$ (100 atmospheres) or at a temperature of from approximately 93°C (200°F) to approximately 149°C (300°F) and a pressure of from atmospheric pressure to $9.8 \times 10^6 N/m^2$ (100 atmospheres).

Cooling and condensing can be supplemented or replaced, if desirable, by another separation process, such as washing with a suitable liquid or absorption on a solid such as with molecular sieves.

The reactor effluent gas, after product separation, is then passed to the next reactor inlet.

The gas leaving the product separator following the final reactor is processed to recover its hydrogen and carbon monoxide content for recycle to a point upstream.

The invention will now be described, by way of example, with reference to the accompanying drawings, in which:

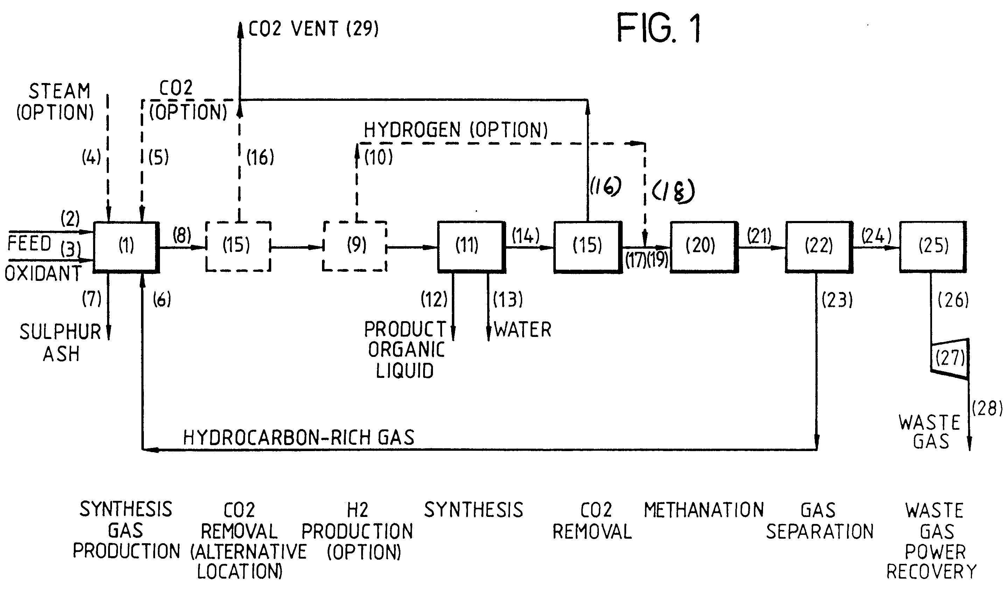

Fig. 1 comprises a flow diagram representing, inter alia, a synthesis gas production unit, a synthesis unit for the production of compounds which are liquids at atmospheric pressure, a carbon dioxide removal unit, a hydrogen bypass a methanator, a gas separator and means for heating a nitrogen rich waste gas either by indirect heat transfer or by direct combustion with an oxidant to give a hot gas which is expanded in an expansion turbine or where such combustion is conducted in a combustion turbine to produce mechanical power before discharge to the atmosphere and

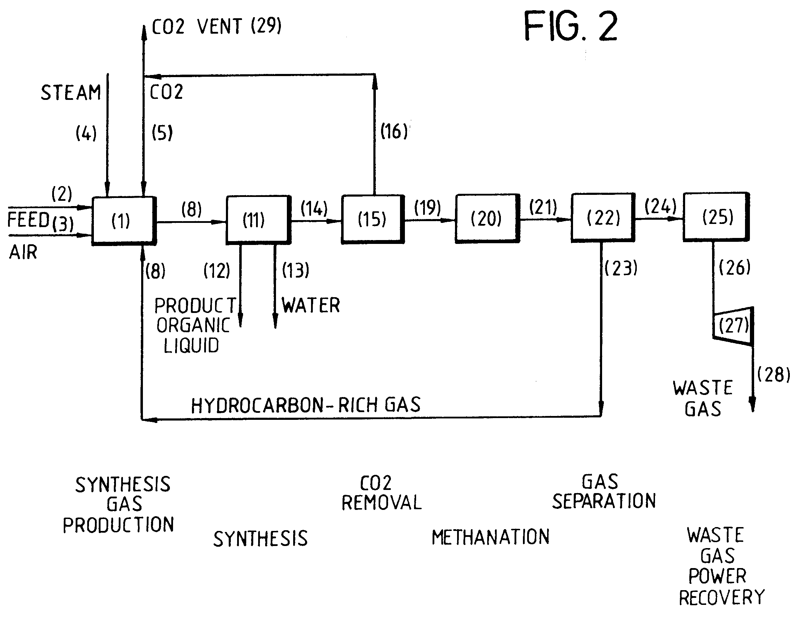

Figure 2 is a flow diagram illustrating the production of octane from methane which, inter alia, does not include a hydrogen bypass.

Referring to the drawings, block 1 of Figure 1 represents a synthesis gas production unit wherein a feedstock 2, which may be a hydrocarbon gas or liquid or a carbonaceous solid such as coal or coke or a mixture of any of these substances, is reacted with a stream of oxygen-containing gas 3 containing at least about 30 molar percent of nitrogen, optional steam 4, optional carbon dioxide 5 and a gas rich in hydrocarbons 6 recycled from downstream as described below. Rejection of sulphur, ash and other waste materials is shown at 7. The product synthesis gas 8 contains hydrogen and carbon monoxide in a molar ratio up to about 4, but more typically about 2, plus the nitrogen content of stream 3. The pressure of the synthesis gas can be up to about $2 \times 10^7 N/m^2$ but usually ranges from about $1 \times 10^6 N/m^2$ to about $4 \times 10^6 N/m^2$.

Block 9 represents an optional step for producing a hydrogen-rich gas stream 10 from the synthesis gas 8, for use downstream as described below. This stream preferably contains at least about 80 molar per cent of hydrogen. Typical arrangements of 9 include a physical separation device such as a membrane or pressure swing absorbers. This facility for by-passing hydrogen around the synthesis unit 11 could be used if passing any additional hydrogen needed for the downstream methanation through the synthesis unit is disadvantageous for the operation of the synthesis unit.

Block 11 represents a synthesis unit for the production of compounds 12 which are liquids at atmospheric pressure and temperature and are composed of carbon, hydrogen and optionally oxygen from the synthesis gas 8. The arrangement of the synthesis reactors within block 11 comprises a series of one or more once-through reactors without recycle and with intermediate removal of product. Any water formed in the synthesis processes is discharged at 13. The tail gas 14 from the synthesis contains unreacted hydrogen and carbon monoxide, carbon dioxide, methane and a high proportion of nitrogen as its main constituents.

Block 15 represents a carbon dioxide removal process, using a solvent such as an amine solution or potassium carbonate solution. The $CO_2$ removed 16 may be recycled in part or wholly to form stream 5. Block 15 may optionally be located upstream of 11.

The scrubbed gas 17 is then optionally mixed with hydrogen rich gas 18 which may be derived from stream 10 and the combined gas stream 19 is the methanated in reactor(s) 20 to achieve essentially complete elimination of carbon oxides by reaction with the hydrogen present. The methanation again comprises reacting carbon monoxide and carbon dioxide in the gas stream with hydrogen to produce methane. The quantity of carbon dioxide removed in 15 and any amount of hydrogen added at 18 are adjusted to give a small excess of hydrogen in the methanator outlet gas 21 where the excess of hydrogen would be up to about 25% of the methanator outlet gas by volume.

Stream 21 is then subjected to a physical separation process 22 which is typically a cryogenic process although an absorptive or diffusional process may be substituted. The products from 22 are principally a hydrocarbon-rich gas stream 23, which is recycled to form stream 206, and a nitrogen-rich waste gas 24.

Stream 24 is typically heated 25 by indirect heat transfer or by direct combustion with an oxidant to give a hot gas 26 which is then expanded in an expansion turbine 27 to produce mechanical power before it is discharged to the atmosphere 28. Stream 21 can also be heated by such direct combustion in turbine 27 which may be a combustion turbine.

The following material balance illustrates the production of octane from methane. Because all the hydrogen need for the methanation stage passes in this example through the synthesis reactors 11, there is no hydrogen by-pass 10, 16 or unit 9. The resulting simplified flow scheme is shown in Figure 2.

The example is based on 1000 kg mole/h of gross methane feed to the synthesis gas production unit (total in streams 2 and 6 = 895.2 + 104.8 kg mole/h).

Other parameters include:

Steam/carbon ratio for synthesis gas production = 1.5,
Outlet temperature from synthesis gas reactor = 950°C,
Conversion of $(H_2 + CO)$ to octane = 90%,
$CO_2$ concentration in stream 19 = 0.5% molar,
Methane concentration in streams 26, 223 = 50%,
Hydrogen concentration in stream 24 = 2%.

**Claims**

1. A process for manufacturing an organic liquid which is methanol, ethanol or a higher alcohol, a Fischer Tropsch product or an olefin, comprising:

    (a) forming a gas comprising hydrogen, nitrogen and carbon monoxide by the partial oxidation of a hydrocarbon or other carbonaceous material with air,

(b) passing the said gas without the removal of the nitrogen through at least one organic liquid synthesis reactor,

(c) condensing and removing the organic liquid to leave a first product gas,

(d) separating carbon dioxide from the first product gas to obtain a second product gas,

(e) methanating the second product gas to obtain a third product gas which is substantially a gas free of carbon oxides, and

(f) separating the hydrocarbons from the third product gas to obtain a nitrogen-rich waste gas and recycling the hydrocarbon rich gas to step a for forming a gas comprising hydrogen, nitrogen and carbon monoxide.

2. A process as claimed in Claim 1 further comprising heating the nitrogen-rich waste gas by indirect heat transfer or by direct combustion with an oxidant to get a hot gas, and expanding that hot gas in a turbine to produce mechanical power before the hot gas is discharged to the atmosphere.

3. A process as claimed in Claim 1 or Claim 2 in which carbon dioxide is additionally separated from the gas of step a.

4. A process as claimed in any preceding claim in which hydrogen formed in step a is partially removed from the gas and combined with said second product gas obtained from step d prior to methanation of the second product gas, the separation of carbon dioxide from the first product gas or from step a along with the hydrogen added to the second product gas obtained in step d being adjusted to give an excess of hydrogen in the third product gas obtained in step e.

5. A process as claimed in Claim 4 in which the separation of carbon dioxide and the addition of hydrogen is adjusted to give a small excess of hydrogen in the third product gas.

6. A process as claimed in Claim 5 in which steam is introduced in step a for forming the said gas comprising hydrogen, nitrogen and carbon monoxide.

7. A process as claimed in any preceding claim in which the carbon dioxide separated from the first product gas in step d or from the gas of step a is introduced into the step a for forming a gas comprising hydrogen, nitrogen and carbon monoxide.

8. A process as claimed in any preceding claim in which the gas from step a is passed through two or more organic liquid synthesis reactors, and organic liquid is condensed and removed from each of the reactors.

9. A process as claimed in Claim 8 in which the organic liquid synthesis reactors operate at a temperature of from 200 to 500°C and a pressure of from atmospheric pressure to $9.8 \times 10^6 N/m^2$ (100 atmospheres) in the presence of a catalyst comprising copper, iron and zinc compounds as the principal active constituents.

10. A process as claimed in Claim 8 or Claim 9 where the condensation process takes place at a temperature of from approximately 93°C (200°F) to approximately 149°C (300°F) and a pressure of from atmospheric pressure to $9.8 \times 10^6 N/m^2$ (100 atmospheres).

**Patentansprüche**

1. Verfahren zur Herstellung einer organischen Flüssigkeit, die Methanol, Äthanol oder ein höherer Alkohol, ein Produkt der Fischer-Tropsch-Synthese oder ein Olefin ist, bei dem man:

(a) durch partielle Oxydation eines Kohlenwasserstoffs oder eines anderen kohlenstoffhaltigen Materials mit Luft ein Gas herstellt, das Wasserstoff, Stickstoff und Kohlenmonoxyd enthält,

(b) das Gas ohne Entfernung des Stickstoffs durch mindestens einen Reaktor zur Synthese einer organischen Flüssigkeit leitet,

(c) die organische Flüssigkeit kondensiert und entfernt, wobei ein erstes Produktgas zurückbleibt,

(d) Kohlendioxyd von dem ersten Produktgas abtrennt, wobei man ein zweites Produktgas erhält,

(e) das zweite Produktgas der Methanisierung unterzieht, um ein drittes Produktgas zu erhalten, das im wesentlichen ein von Kohlenoxyden freies Gas ist, und

(f) die Kohlenwasserstoffe von dem dritten Produktgas abtrennt, um ein stickstoffreiches Abgas zu erhalten, und das kohlenwasserstoffreiche Gas in die Stufe (a) zur Erzeugung eines Gases, das Wasserstoff, Stickstoff und Kohlenmonoxyd enthält, zurückführt.

2. Verfahren gemäß Anspruch 1, wobei man außerdem das stickstoffreiche Abgas durch indirekte Wärmeübertragung oder durch direkte Verbrennung mit einem Oxydationsmittel erhitzt, um ein heißes Gas zu erzielen, und daß man das heiße Gas in einer Turbine zur Erzeugung mechanischer Energie expandieren läßt, bevor das heiße Gas in die Atmosphäre abgelassen wird.

**3.** Verfahren gemäß Anspruch 1 oder 2, wobei Kohlendioxyd zusätzlich aus dem Gas von Stufe (a) abgetrennt wird.

**4.** Verfahren gemäß einem der vorhergehenden Ansprüche, wobei man Wasserstoff, der in Stufe (a) gebildet worden ist, teilweise aus dem Gas entfernt und mit dem zweiten Produktgas, das aus Stufe (d) erhalten worden ist vor der Methanisierung des zweiten Produktgases vereinigt, wobei die Abtrennung von Kohlendioxyd aus dem ersten Produktgas oder aus dem Gas von Stufe (a) zusammen mit der Zuführung von Wasserstoff zu dem zweiten Produktgas, das in Stufe (d) erhalten wird, derart eingestellt wird, daß man in dem dritten Produktgas, das in Stufe (e) erhalten wird, einen Wasserstoffüberschuß erhält.

**5.** Verfahren gemäß Anspruch 4, wobei die Abtrennung von Kohlendioxyd und die Zuführung von Wasserstoff derart eingestellt wird, daß man in dem dritten Produktgas einen geringen Überschuß an Wasserstoff erhält.

**6.** Verfahren gemäß Anspruch 5, wobei Wasserdampf in Stufe (a) zur Herstellung des Gases aus Wasserstoff, Stickstoff und Kohlenmonoxyd eingeführt wird.

**7.** Verfahren gemäß einem der vorhergehenden Ansprüche, wobei man das Kohlendioxyd, das aus dem ersten Produktgas in Stufe (d) oder aus dem Gas von Stufe (a) abgetrennt wird, in die Stufe (a) zur Herstellung eines Gases, das Wasserstoff, Stickstoff und Kohlenmonoxyd enthält, einführt.

**8.** Verfahren gemäß einem der vorhergehenden Ansprüche, wobei man das Gas aus Stufe (a) durch zwei oder mehrere Reaktoren zur Synthese einer organischen Flüssigkeit leitet und organische Flüssigkeit aus jedem dieser Reaktoren kondensiert und abtrennt.

**9.** Verfahren gemäß Anspruch 8, wobei die Reaktoren zur Synthese einer organischen Flüssigkeit bei einer Temperatur von 200 bis 500°C und einem Druck von Atmosphärendruck bis $9,8 \times 10^6 N/m^2$ (100 Atmosphären) in Gegenwart eines Katalysators, der als hauptsächliche aktive Bestandteile Kupfer-, Eisen- und Zinkverbindungen enthält, betrieben werden.

**10.** Verfahren gemäß Anspruch 8 oder 9, wobei der Kondensationsvorgang bei einer Temperatur von etwa 93°C (200°F) bis etwa 149°C (300°F) und einen Druck von Atmosphärendruck bis $9,8 \times 10^6 N/m^2$ (100 Atmosphären) stattfindet.

**Revendications**

**1.** Procédé de préparation d'un liquide organique qui est le méthanol, l'éthanol ou un alcool supérieur, un produit de Fischer-Tropsch ou une oléfine, comprenant :

(a) la formation d'un gaz comprenant de l'hydrogène, de l'azote et du monoxyde de carbone par oxydation partielle d'un hydrocarbure ou d'autre matière carbonée par de l'air,
(b) le passage dudit gaz, sans l'élimination de l'azote, à travers au moins un réacteur de synthèse de liquide organique,
(c) la condensation et séparation du liquide organique pour laisser un premier produit gazeux,
(d) la séparation du dioxyde de carbone contenu dans le premier produit gazeux pour obtenir un deuxième produit gazeux,
(e) la méthanisation du deuxième produit gazeux pour obtenir un troisième produit gazeux qui est essentiellement un gaz exempt d'oxydes de carbone, et
(f) la séparation des hydrocarbures contenus dans le troisième produit gazeux pour obtenir un gaz résiduaire riche en azote et le recyclage du gaz riche en hydrocarbure vers l'étape (a) pour former un gaz comprenant de l'hydrogène, de l'azote et du monoxyde de carbone.

**2.** Procédé selon la revendication 1, comprenant en outre le chauffage du gaz résiduaire riche en azote par transfert thermique indirect ou par combustion directe avec un oxydant pour obtenir un gaz chaud, et l'expansion de ce gaz chaud dans une turbine pour générer une force mécanique avant décharge du gaz chaud dans l'atmosphère.

**3.** Procédé selon la revendication 1 ou 2, dans lequel le dioxyde de carbone est en plus séparé du gaz de l'étape (a).

**4.** Procédé selon l'une quelconque des revendications qui précèdent, dans lequel l'hydrogène formé dans l'étape (a) est partiellement séparé du gaz et combiné avec ledit deuxième produit gazeux obtenu à l'issue de l'étape (d) avant méthanisation du deuxième produit gazeux, la séparation du dioxyde de carbone contenu dans le premier produit gazeux ou provenant de l'étape (a) ainsi que l'hydrogène ajouté au deuxième produit gazeux obtenu à l'issue de l'étape (d) étant ajustés pour fournir un excédent d'hydrogène dans le troisième produit gazeux obtenu à l'issue de l'étape (e).

**5.** Procédé selon la revendication 4, dans lequel la séparation du dioxyde de carbone et l'addition de l'hydrogène sont ajustées pour fournir un petit excédent d'hydrogène dans le troisième produit gazeux.

6. Procédé selon la revendication 5, dans lequel de la vapeur d'eau est introduite dans l'étape (a) pour former ledit gaz comprenant de l'hydrogène, de l'azote et du monoxyde de carbone.

7. Procédé selon l'une quelconque des revendications qui précèdent, dans lequel le dioxyde de carbone séparé du premier produit gazeux dans l'étape (d) ou provenant du gaz de l'étape (a) est introduit dans l'étape (a) pour former un gaz comprenant de l'hydrogène, de l'azote et du monoxyde de carbone.

8. Procédé selon l'une quelconque des revendications qui précèdent, dans lequel le gaz obtenu à l'issue de l'étape (a) est passé par deux réacteurs de synthèse de liquides organiques, ou davantage, et le liquide organique est condensé et séparé à partir de chacun des réacteurs.

9. Procédé selon la revendication 8, dans lequel les réacteurs de synthèse de liquides organiques opèrent à une température de 200 à 500°C et à une pression située dans la plage allant de la pression atmosphérique à $9,8 \times 10^6$ N/m$^2$ (100 atmosphères) en présence d'un catalyseur comprenant des composés de cuivre, de fer et de zinc, à titre de principaux constituants actifs.

10. Procédé selon la revendication 8 ou la revendication 9, dans lequel le procédé de condensation est effectué à une température d'environ 93°C (200°F) à environ 149°C (300°F) et une pression située dans la plage allant de la pression atmosphérique à $9,8 \times 10^6$ N/m$^2$ (100 atmosphères).

FIG. 1

CO2 VENT (29)

STEAM (OPTION)

CO2 (OPTION)

HYDROGEN (OPTION)

(4)    (5)         (16)         (10)              (16)  (18)

(2)

FEED  (3)
OXIDANT

(1)    (8)    (15)    (9)    (11)    (14)    (15)    (17)(19)    (20)    (21)    (22)    (24)    (25)

(7)    (6)                    (12)    (13)                                    (23)    (26)

SULPHUR
ASH

PRODUCT         WATER
ORGANIC
LIQUID

(27)

(28)

HYDROCARBON-RICH GAS                                            WASTE
GAS

SYNTHESIS      CO2         H2         SYNTHESIS    CO2      METHANATION    GAS        WASTE
GAS         REMOVAL    PRODUCTION                REMOVAL                 SEPARATION   GAS
PRODUCTION  (ALTERNATIVE  (OPTION)                                                    POWER
LOCATION)                                                                   RECOVERY

EP 0 601 886 B1

8

FIG. 2

STEAM (4)

CO2 VENT (29)

CO2 (5)

FEED (2)

AIR (3)

(8)

(1)

(8)

PRODUCT ORGANIC LIQUID (12)

WATER (13)

(14)

(11)

(15)

(16)

(19)

(20)

(21)

(22)

(23)

HYDROCARBON-RICH GAS

(24)

(25)

(26)

(27)

WASTE GAS (28)

SYNTHESIS GAS PRODUCTION

SYNTHESIS

CO2 REMOVAL

METHANATION

GAS SEPARATION

WASTE GAS POWER RECOVERY